Europäisches Patentamt

European Patent Office

Office européen des brevets

(11) Publication number: **0 233 578**
A2

(12) # EUROPEAN PATENT APPLICATION

(21) Application number: **87101839.6**

(22) Date of filing: **10.02.87**

(51) Int. Cl.³: **C 12 N 15/00**
C 12 P 21/02, C 12 N 1/20
A 61 K 37/02
//(C12N1/20, C12R1:19)

(30) Priority: **10.02.86 JP 27593/86**

(43) Date of publication of application:
**26.08.87** Bulletin **87/35**

(84) Designated Contracting States:
**CH DE ES FR IT LI NL SE**

(71) Applicant: OTSUKA PHARMACEUTICAL CO., LTD.
9, Kandatsukasacho 2-chome
Chiyoda-ku Tokyo 101(JP)

(72) Inventor: Nishida, Tsutomu
240-118, Ooshiro Otsu-cho
Narutoshi-shi Tokushima 772(JP)

(72) Inventor: Takano, Masaaki 57-1, Aza-hachigami
Haitsu-datemusume B207 Sasakino Matsushige-cho
Itano-gun Tokushima 771-02(JP)

(72) Inventor: Nishino, Naoki
33-5, Myojin-cho 1-chome
Tokushima-shi Tokushima 770(JP)

(72) Inventor: Ohmoto, Yasukazu
35-6, Aza-hachisimo Sasakino Matsushige-cho
Itano-gun Tokushima 771-02(JP)

(72) Inventor: Ishigami, Fumitsugu
1-30, Aza-nivu Shinkirai Kitajima-cho
Itano-gun Tokushima 771-02(JP)

(72) Inventor: Hirai, Yoshikatsu
5-49, Aza-ekogawa Shinkirai Kitajima-cho
Itano-gun Tokushima 771-02(JP)

(74) Representative: Hansen, Bernd, Dr.rer.nat. et al,
Hoffmann, Eitle & Partner Patentanwälte Arabellastrasse
4
D-8000 München 81(DE)

(54) Polypeptide possessing interleukin-2 activities.

(57) A polypeptide possessing interleukin-2 activities having
an amino acid sequence in which at least one amino acid out
of the second to eleventh and 128th to 133rd amino acid
sequences from the N terminal (Ala) of amino acid sequence
of human interleukin-2 and the substance having similar effects is deleted or replaced, and processes for preparing the
same.

EP 0 233 578 A2

## Polypeptide Possessing Interleukin-2 Activities

### Technical Field

This invention relates to a novel polypeptide possessing interleukin-2 (hereinafter abbreviated as IL-2) activities, and more particularly to IL-2 derivatives low in antigenicity and suited to use in pharmaceuticals being composed of IL-2 active polypeptide portion on the IL-2 molecule which is a biopolymer substance produced from human lymphocyte cells.

### Background Arts

IL-2 is a lymphokine being produced, for example, by stimulating human lymphocytes with phytohemagglutinin (PHA), concanavalin A (Con A), pokeweed mitogen (PWM) or other mitogen, and antigenic substances (Smith, K. A. et al., Adv. Immunol, 31, PP 37 1981).

This IL-2 is an extremely important biological matter for the proliferation or differentiation of lymphocytes and activation of biological defence mechanism (Gills, S. et al., Immonol. Rev., 63, PP 166, 1982), and it is known to present, for example, such physiological activities as proliferation of T-lymphocyte cells (Ruscetti, F. W. et al., J. Immunol., 119, PP 131, 1977; Smith, K.A., Immunol. Rev., 51, PP. 337, 1980), induction of cytotoxic T lymphocytes (Wagner, H. et al., Immonol. Rev., 51, PP 215, 1980), activation of natural killer cells (Henney, C.S. et al., Nature, 291, PP 335, 1981), induction of lymphokine activated killer cells (Grinm, E.A. et al., J. Exp. Med., 155, PP 1823, 1982), production of γ-type interferon (Farrar, J. J. et al., Immonol. Rev., 63, PP 129, 1982), and stimulation of antibody producing cells, (Waldmann, T. A. et al., J. Exp. Med., 160, PP 1450, 1984). Such IL-2 and substances having similar effects and methods of preparation thereof have been disclosed, for example, in the U.S. Patent No. 4,518,584, PCT International Publication No. WO85/00817.

Because of said physiological activities, IL-2 is expected to cure the cancer in a patient of cancer disturbed in the biological defense system by stimulating the lymphocyte cells to protect the patient from various infections, while simultaneously activating the cell groups that attack cancer cells. Furthermore, for the sake of the action to stimulate the immune system, it is also considered to be effective for the treatment of immune deficiency diseases such as AIDS, lupus, multiple sclerosis and rheumatoid arthritis.

Analysis of the role of IL-2 in the tumor immune response system had been conventionally studied by using natural IL-2 produced by stimulating peripheral blood mononuclear cells, palatotonsillar lymph tissues, leukemia-derived cells or others by mitogen or the like, but more recently, using IL-2 produced by the gene recombinant technology, it has been gradually attempted to test the immunity intensifying activity and pharmacological effects and apply into therapy of cancers.

At the present, when mass supply of IL-2 into clinical use is realized by the development of said recombinant IL-2, various cancer treatments are attempted at many institutes, and these attempts may be roughly classified into (1) direct administration of IL-2 to the patient, (2) transfer of lymphocytes activated in vitro by IL-2 into the patient, and (3) combined use of transfer of lymphocytes activated in vitro by IL-2 into the patient and direct administration of IL-2 to the patient. In any case, however, increase of dose of IL-2 and prolongation of term of administration may be supposed, and in such long-term treatment, it is necessary to avoid appearance of antibodies to IL-2, which is lately regarded as an important problem to be considered clinically.

For example, what was most expected in the realization of human insulin was the belief that antibodies would not be produced in the human body, but actually among the patients treated with human insulin, detections of insulin antibodies were reported in about one-third of them in a year (Fireman, P., Fineberg, S. E. and Galloway, J. A. Diabetes Care, 5, suppl. 2, PP 119, 1982). This was the first study to report that the human insulin comes to have its antigenicity in humans by long-term use of human insulin. Similar appearances of antibodies were also reported in patients treated with human growth hormone for a long period (Hint, R. L. et al., Lancet, PP 1276, 1982; Shizume, K., Human growth hormone, J. of Japan Society of Pharmacists, 34, suppl. No. 11). Besides, appearance of neutral antibodies is also reported in the patients treated a long time with interferon produced not only from gene recombinants but also from human normal fibroblast cells (Angelika, V. et al., Nature, 289, PP 496, 1981).

Estimating from these reports, it is predicted that antibodies to IL-2 may appear if IL-2 be administered for a long term, and it is considered that the therapeutic effect of IL-2 be extremely weakened if an antibody recognizing the active portion of IL-2 or its proximal portion should appear in the patient.

Disclosure of the Invention

It is hence a primary object of this invention to present a novel polypeptide possessing IL-2 activities. More particularly, this invention is intended to present a novel peptide which possesses lower the possiblity of

- 5 -

0233578

appearance of neutral antibody which recognizes the vicinity of IL-2 active site to impede the activity, and also possesses the IL-2 activities, by deleting or replacing as much as possible the parts not responsible for IL-2 activities out of the amino acid sequence of known IL-2.

It is another object of this invention to present a mass-producing technology of such novel IL-2 active polypeptide by a genetic engineering technique.

It is a further object of this invention to present pharmaceutical preparations containing said polypeptide preferable clinically.

The above objects and other features of this invention are described herein.

This invention presents a polypeptide possessing IL-2 activities characterized by an amino acid sequence in which at least one amino acid out of the second to the eleventh, and the 128th to the 133rd amino acid sequences from the N terminal (Ala) of amino acid sequence of the human IL-2 represented by the formula (1)

1

Ala - Pro - Thr - Ser - Ser - Ser - Thr - Lys - Lys -

10

Thr - Gln - Leu - Gln - Leu - Glu - His - Leu - Leu -

20

Leu - Asp - Leu - Gln - Met - Ile - Leu - Asn - Gly -

Ile - Asn - Asn - Tyr - Lys - Asn - Pro - Lys - Leu -

Thr - Arg - Met - Leu - Thr - Phe - Lys - Phe - Tyr -

Met - Pro - Lys - Lys - Ala - Thr - Glu - Leu - Lys -

His - Leu - Gln - Cys - Leu - Glu - Glu - Glu - Leu -

Lys - Pro - Leu - Glu - Glu - Val - Leu - Asn - Leu -

Ala - Gln - Ser - Lys - Asn - Phe - His - Leu - Arg -

Pro - Arg - Asp - Leu - Ile - Ser - Asn - Ile - Asn -

Val - Ile - Val - Leu - Glu - Leu - Lys - Gly - Ser -

Glu - Thr - Thr - Phe - Met - Cys - Glu - Tyr - Ala -

Asp - Glu - Thr - Ala - Thr - Ile - Val - Glu - Phe -

Leu - Asn - Arg - Trp - Ile - Thr - Phe - Cys - Gln -

Ser - Ile - Ile - Ser - Thr - Leu - Thr        ------ (1)

and substances having similar effects is deleted or replaced.

The present inventors, after strenuously accumulating studies, prepared monoclonal antibodies which can identify various positions of a known human IL-2, and obtained a neutral antibody that impedes the IL-2 activity, investigated with which position of the amino acid sequence of human IL-2 is this neutral antibody coupled, estimated the active site of human IL-2, and also estimated the position of amino acid sequence of IL-2 responsible for the binding with the IL-2 receptor existing on a cell membrane which is considered to be necessary for the expression of activity of human IL-2. The existence of said IL-2 receptor is as reported by W. J. Leonard et al. (Science, 230, PP 633, 1985). As a result, among the IL-2 derivatives the inventors produced by the genetic engineering technology, the polypeptide possessing the first to 58th amino acid sequence from the N terminal of the amino acid sequence of the formula (1), the polypeptide possessing the first to 100th amino acid sequence from said N terminal, the polypeptide possessing the first to 122nd amino acid sequence from said N terminal, and the polypeptide possessing the 60th to 133rd amino acid sequence from said N terminal did not possess IL-2 activities, while the polypeptide possessing the first and 12th to 133rd amino acid sequence from said N terminal and the polypeptide possessing the first to 127th amino acid sequence from said N terminal were found to possess IL-2 activities.

On the basis of the above results of study, the inventors further accumulated researches, and found out that the second to eleventh amino acid sequence from the N terminal of amino acid sequence of a known human IL-2, that is, Pro-Thr-Ser-Ser-Ser-Thr-Lys-Lys-Thr-Gln, and the 128th to 133rd amino acid sequence from said N terminal, that is, Ile-Ile-Ser-Thr-Leu-Thr are not substantially responsible for the expression of IL-2 activities and binding with IL-2 receptor, and that an intended polypeptide possessing IL-2 activities and capable of lowering the possibility of appearance of neutral antibody could be obtained by deleting or arbitrarily replacing at least part of these amino acid sequence. This invention is achieved on the basis of this knowledge.

In this Specification, the amino acids in polypeptide and others are expressed according to the abbreviations in the amino acid nomenclature specified by the IUPAC and the naming method of amino acid residual groups by abbreviations customarily used in this field, and the same principle applies to the expression of nucleic acid in the base sequence.

Incidentally, in this Specification, human IL-2 is expressed as a polypeptide possessing amino acid sequence shown in the formula (1), and this human IL-2 is supposed to include also substances having effects similar to those of the human IL-2, for example, a polypeptide having the 125th Cys of the formula (1) replaced by other amino acid such as Ser and Ala, or a polypeptide keeping the total amino acid sequence of the formula (1) and also having one or more amino acids at its N terminal or C terminal such as a polypeptide having Met coupled with the terminal of formula (1), etc. Therefore, the IL-2 active polypeptide in this invention also includes those obtained by deleting or replacing at least part of said specific portions from the IL-2 similar effects substances possessing these amino acid sequence. Here, the other amino acids that can be replaced may be any $\alpha$-amino acid to compose the human protein and are not particularly limited, but since Cys may form an intramolecular or intermolecular bonding based on its SH group, and considering this point, such amino acids may be preferably any other amino acids than Cys.

Hereinafter the polypeptide of this invention is further described from the standpoint of its method of preparation.

The polypeptide of this invention may be prepared, for example by a genetic engineering technique, by using a gene to encode the polypeptide, incorporating it into the vector of microorganism, and replicating, transcribing and translating it within the cell of this microorganism.

The gene used in the above method may be fully synthesized by chemical synthesis of nucleic acid according to the conventional method such as triester method (Nature, 310, PP 105, 1984). Or when using a gene encoding a known human IL-2, it may be easily prepared by deleting or modifying the codon of the nucleic acid sequence to encode said specific amino acid sequence from this gene, according to the conventional method including the above means of chemical synthesis. In this process, selection of genetic code corresponding to a desired amino acid may be arbitrary, and for example, the conventional method considering the frequency of use of the codon of the host to be utilized may be employed (Nucl. Acid Res., 9, PP 43-74, 1981, etc.).

Practical examples of the gene to encode said IL-2 that can be utilized in the preparation of polypeptide of this invention may include those possessing plasmid pHIG5-3 having human IL-2 cDNA isolated from human tonsilar cDNA library (Maeda, S. et al., Biochem. Biophys. Res. Comm., 115, p. 1040, 1983) and genes manufactured according to the methods mentioned in the published literature (Science, 224, PP 1431, 1984; id., 223, PP 1412, 1984; Nucleic Acid Res., 11, PP 4307, 1983, etc.).

Deletion or modification of desired codon from the nucleic acid sequence possessed by said gene may be executed depending on the amino acid sequence of the intended polypeptide, and in its operation, cleavage, coupling and phosphorylation of DNA may be effected according to the conventional method of processing of various intended enzymes such as restriction enzyme, DNA ligase, polynucleotide kinase, and DNA polymelase. Besides, the DNA fragments or synthetic linkers for encoding the desired amino acid sequence may be easily manufactured by said chemical synthesis. For this operation, moreover, other methods may be also used, such as the site-specific mutagenesis using a primer composed of synthetic oligonucleotide of about 15 to 30-mer for encoding a desired modification as in conventional process (Proc. Natl. Acad. Sci., 81, PP 5662-5666, 1984).

The process of isolation and purification of gene and nucleic acid in the execution of the above operation may conform, for example, to the conventional method such as agarose electrophoresis, and the obtained gene may be replicated by an ordinary method of using a vector as partly described later.

The desired gene obtained by the above method may be determined and identified in its base sequence by the chemical modification method proposed by Maxam and Gilbert

(Maxam-Gilbert, Meth. Enzym., 65, PP 499-560, 1980) or the dideoxinucleotide chain termination method using M13 phage (Messing, J. and Vielra J., Gene, 19, PP 269-276, 1982).

Practical examples of operation and method suggested above will be later described in Examples, but the method is not particularly limited, but any method widely known in the field may be applied.

By using thus obtained gene to encode the polypeptide of this invention (called the gene of this invention here-inafter), the polypeptide of this invention may be produced in a large quantity by the gene recombinant technology. This invention is also intended to present such novel gene and a manufacturing technology of the polypeptide of this invention making use of this gene.

The polypeptide of this invention may be manufactured by following a general conventionally known gene recombinant technology except that said specific gene is used. This technology may, for example, conform to the method applied in the production of human IL-2 (Science, 224, PP 1431, 1984; Biochem. Biophys. Res. Comm., 130, PP 692, 1985; Proc. Natl. Acad. Sci. U.S.A., 80, PP 5990, 1983; etc.). More specifically, by preparing a recombinant DNA allowing the gene of this invention to express in the host's cells, it is introduced into the host's cells to transform, and this transformant is cultivated.

As the host's cells, either eucaryote or procaryote may be used. Cells of eucaryote include those of vertebrates, yeast, etc., and as the cells of vertebrates, Cos cells from monkey (Gluzman, Y., Cell, 23, PP 175-182, 1981) and dihydrofolic acid reductase deficiency strains of Chinese hamster ovarian cells (Urlaug, G. et al., Proc. Natl. Acad. Sci., U.S.A., 77, PP 216-422, 1980) are often used, but the usable cells are not limited to them. As the expression vector of vertebrate cells, those possessing promotor located upstream of the gene to be expressed, RNA splicing site, polyadenylation site or transcription termination sequence may be used, and if necessary replication origin may be possessed. As a practical example of this expression vector, p SV 2dhfr having an initial promotor of SV40 (S. Subramani, R. Mulligan and P. Berg, Mol. Cell, Biol, 1 (9), PP 854-864) may be presented, but others may be also used.

As the eucaryotic microorganism, the yeasts are generally used, and above all Saccharomyces may be effectively utilized. As the expression vector of eurcaryotic microorganism such as yeast, for example, p AM 82 having a promoter to the acid phosphatase gene (Miyanohara, A. et al., Proc. Natl. Acad. Sci., U.S.A., 80, PP 1-5, 1983) may be preferably used.

As the host of procaryote, Escherichia coli and Bacillus subtilis are generally known well. In this invention, for example, by using a plasmid vector that can be replicated within the host bacteria, an expression plasmid provided with promotor and SD (Shine and Dalgarno) base sequence in the upstream of this gene and ATG necessary for initiating protein synthesis may be used so that the gene of this invention may express in this vector. As the E. coli as the host bacteria, the E. coli K12 strain is widely used, but other known strains and vectors may be also used. As the promotor, for example, tryptophan promotor, PL promotor, lac promotor, lpp promotor and others may be used, and any one of these can express the gene of this invention.

Referring now to an example of using tryptophan promotor, the process may be further described. Using tryptophan promotor and vector p TM1 having SD sequence as the expression vector (Imamoto, F., Metabolism, 22, PP 289, 1985), the gene to encode the polypeptide of this invention, provided with ATG if necessary, may be linked to the site of restriction enzyme ClaI existing downstream of the SD sequence. Incidentally, instead of direct expression system, the process may also depend on a fused protein expression system, for example, using β-galactosidase or β-lactamase.

As the method of introduction of thus obtained expression vector into the host's cell and transformation by it, general methods, such as the process of collecting cells mainly in the logarithmic proliferative stage, treating them with $CaCl_2$ so as to be in a state ready to take in DNA spontaneously, and taking in the vector, may be employed. In this method, $MgCl_2$ or $RbCl$ may be coexistent, as is usually known, in order to further enhance the efficiency of transformation. Or the method of transforming after first transforming host's cells into spheroplast or protoplast can also be employed.

The desired transformat strain thus obtained can be cultivated by a conventional method, and by this culture, the polypeptide of this invention may be produced and accumulated. As the culture medium used in this cultivation, any medium commonly used in ordinary cell incubation may be used, of which practical examples may include L medium, E medium, M9 medium, and other known media containing various carbon source, nitrogen source, inorganic salt, vitamins, and others. When said tryptophan promotor is used, it is possible to incubate by using a culture medium containing casamino acids in order to encourage the action of promotor, such as M9 minimum medium, and it may be also possible to add chemical agents such as indole—acrylic acid to the culture medium at a proper timing of incubation in order to intensify the action of the tryptophan promotor.

Thus, the polypeptide of this invention produced inside or outside of the cell of the recombinant microorganism is separated by a conventional method, and may be isolated and purified by various operations depending on its physical and chemical properties. These operations themselves may be realized by combining known means properly, and practical examples may include the method of making use of difference in solubility such as salting-out by using ordinary protein sedimentation agent and solvent sedimentation method, the method making use of difference in molecular weight mainly such as dialysis, ultrafiltration, gel filtration, and SDS-polyacrylamide gel electrophoresis, the method making use of difference in electric charge such as ion exchange chromatography, the method making use of specific affinity such as affinity chromatography, the method making use of difference in hydrophobic property such as reverse phase high performance liquid chromatography, the method making use of isoelectric point such as isoelectric electrophoresis, and their combinations.

Thus obtained polypeptide of this invention may be freeze-dried if necessary. In such a case, stabilizing agents such as sorbitol, mannitol, dextrose, maltose, glycerol, and human serum albumin (HSA) may be added.

## Brief Description of the Drawings

Fig. 1 is a diagram to show the process of building up plasmid p ATm IL-2-1 and plasmid p ATm IL-2-2 from plasmid p HJG5-3 and plasmid p AT153, and the features of the obtained plasmids;

Fig. 2 is a diagram to show the process of building up plasmid p trp IL-2D2-11 from plasmid p ATm IL-2-2 and plasmid p TM1, and the features of the obtained plasmid; and

Fig. 3 is a diagram to show the process of building up plasmid p trp IL-2D8 from plasmid p ATm IL-2-1 and plasmid p TM1, and also plasmid p trp IL-2D8Tlpp and plasmid p trp IL-2Bal from said plasmid p trp IL-2D8 and plasmid p INIIIomp Al, and the features of the obtained plasmids.

## Industrial Applicability

The polypeptide of this invention possesses IL-2 activities, and is useful as a pharmaceutical preparation as stated above. Furthermore, this polypeptide is low in toxicity, which is very advantageous for said application.

The polypeptide of this invention may present a useful pharmaceutical preparation for said various medical applications when used as active ingredient. The pharmaceutical preparation is administered by various routes depending on the dosage form, with the effective amount of the polyeptide of this invention adjusted together with pharmaceutically acceptable ordinary nontoxic carriers in the form of various pharmaceutical compositions being contained. The dosage form is not particularly defined as far as the polypeptide of this invention is effectively contained, and solid forms such as tablet, powder and granule may be applied, but usually, the liquid forms such as solution, suspension and emulsion are preferable. When forming into solution, emulsion or suspension, any diluent commonly used in this field may be used, such as water, ethyl alcohol, propylene glycol, ethoxide isostearylalcohol, polyoxide isostearlylalcohol, polyoxyethylene sorbitan fatty acid ester, and others. In this case, salt, glucose or glycerin in a sufficient quantity for preparing an isotonic solution may be contained in the pharmaceutical preparation. Or it is also possible to present in a form of dry product that may be changed into a liquid form before use by adding proper ordinary carrier. The obtained pharmaceutical preparations are administered by the route depending on the dosage form, for example, the injection preparation is administered intravenously, intramuscularly, subcutaneously, intradermally or peritoneally, while solid preparations may be administered orally or parenterally. The dose of the pharmaceutical preparation varies depending on the dosage form, route of administration, purpose of use, and age, sex or degree of disease of the patient, but usually it is preferable to administer a daily dose of about 0.01 to 1 mg/kg as the effective ingredient in a single dose or several divided portions. Moreover, the polypeptide of this invention may be applied in a therapy in which lymphocytes are cultivated in its presence, and killer cells showing cell-disturbing property to tumor cells are induced, and they are returned into the body of the patient of cancer.

The polypeptide presented by this invention possesses IL-2 activities, and is very useful, for example, as the remedy for cancer or immune deficiency based on such activities.

In addition, the polypeptide of this invention is, as compared with the existing human IL-2, low in the possibility of appearance of neutral antibody and safe enough to realize long-term treatment. Furthermore, by its application, the risk of the neutral antibodies to impede the IL-2 produced by lymphocytes of the patients may be lowered. Therefore, it is extremely useful clinically. Moreover, the polypeptide of this invention is advantageous in that it can be used effectively even in patients having already neutral antibodies as a result of treatment by existing IL-2.

Examples

Examples are illustrated below with the purpose of describing this invention in greater details, but they are not intended to limit the scope of this invention.

In the Examples mentioned below, meanwhile, the IL-2 activities were measured by using the IL-2 dependent mouse T-cells (CTLL-2) following the method proposed by Gillis and Smith (Gillis, S. and Smith, K. A., J. Immunol., 120, PP 2027, 1978).

## Example 1

(1)   Construction of plasmid p ATm IL-2-2

The IL-2 cDNA plasmid p HIG5-3 isolated from human tonsillar cDNA library (Maeda, S. et al., Biochem. Biophys. Res. Comm., 115, PP 1040, 1983) was cleaved by restriction enzymes Hind III and Pvu II, and 1.2 kilobase (kb) fragment containing IL-2 cDNA was isolated and refined by agarose gel electrophoresis.

This DNA fragment was further cleaved by a restriction enzyme HgiAI, and 0.8 kb of DNA fragment to encode the mature protein of IL-2 was isolated and refined by agarose gel electrophoresis, and the cleavage site by restriction enzyme HgiAI was treated with $T_4$ DNA polymerase to form a blunt end. To the obtained DNA fragment, synthetic oligonucleotides [5'd ($_{OH}$CGATAATGGCA$_{OH}$) 3'] and [5'd ($_p$TGCCATTAT$_{OH}$) 3'] obtained by phosphorylating 5' terminal

were linked by $T_4$ DNA ligase, and this DNA fragment was isolated and refined by agarose gel electrophoresis.

On the other hand, plasmid pAT153 (Nature, 283, PP 216, 1980) was cleaved by a restriction enzyme ClaI, and at this cleavage site the DNA fragment linked with the synthetic oligonucletide obtained above was linked by using $T_4$ DNA ligase, and by using this, the E. coli HB101 strain was transformed, and E. coli HB101/pATm IL-2-1 containing a desired plasmid p ATm IL-2-1 (4.5 kb) was obtained. This E. coli HB101/pATm IL-2-1 was deposited, as Escherichia coli BH101/p ATm IL-2-1 (FERM BP-1256), in the Fermentation Research Institute Agency of Industrial Science and Technology, No. 1-3, Higashi 1 chome Yatabe-machi, Tsukuba-gun, Ibaraki-ken, Japan, on 23 January, 1987.

The obtained plasmid p ATm IL-2-1 was cleaved by restriction enzymes BanI and ClaI, and 0.8 kb of BanI-ClaI DNA fragment was isolated and purified by agarose gel electrophoresis, and the both ends of this fragment were treated with DNA polymerase I (Klenow fragment) to form blunt ends.

On the other hand, the 5' terminal of ClaI linker [5'd ($_{HO}$CATCGATG$_{OH}$)3', made by Takara Shuzo Co., Ltd. (Japan)] was phosphorylated by $T_4$ polynucleotide kinase, and was linked to said DNA fragment having blunt ends at the both terminals, by $T_4$ DNA ligase, and was cleaved by restriction enzyme ClaI, and 0.8 kb of ClaI-ClaI DNA fragment was obtained by agarose gel electrophoresis.

Besides, after cleaving the plasmid pAT153 by restriction enzyme ClaI, 0.8 kb of ClaI-ClaI DNA fragment obtained above was linked to this cleavage site by $T_4$ DNA ligase, and the E. coli HB101 strain was transformed by this linkage, and E. coli HB101/p ATm IL-2-2 containing a desired plasmid pATm IL-2-2 was obtained.

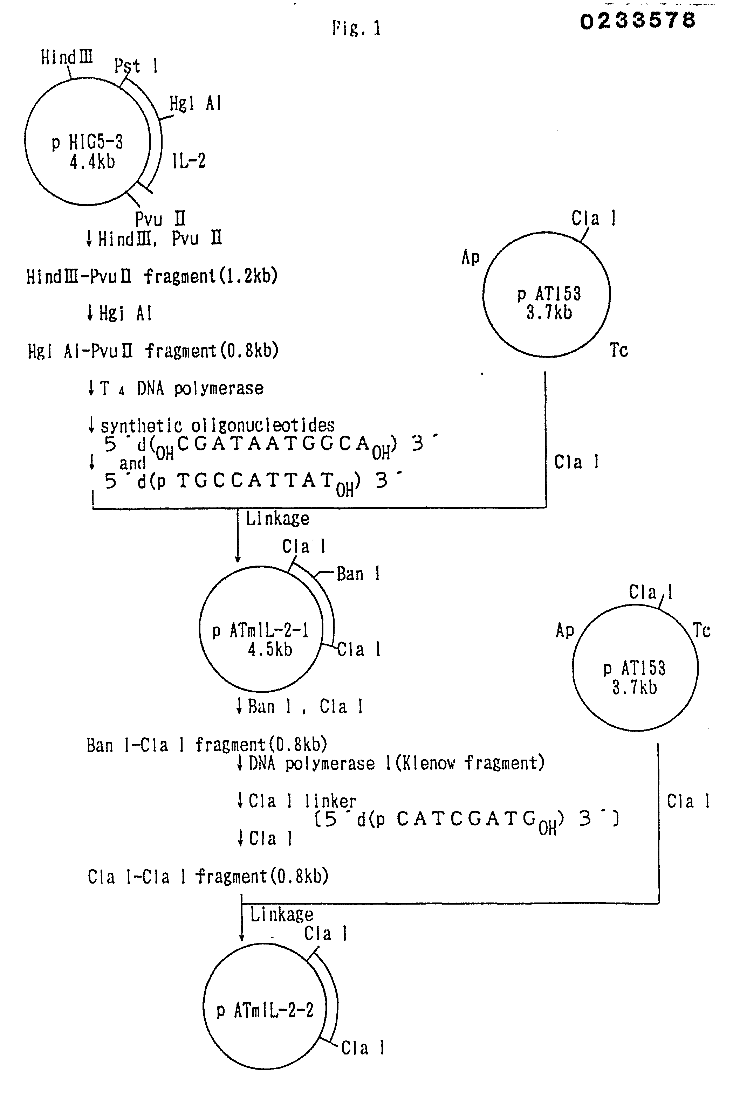

The outline of this process is shown in Fig. 1.

(2)  Construction of plasmid p trp IL-2D2-11

Plasmid p ATm IL-2-2 was cleaved by restriction enzymes ClaI and StuI, and 0.44 kb of ClaI-StuI DNA fragment for encoding the mature protein of IL-2 was isolated and purified by agarose gel electrophoresis, and it was partially cleaved by a restriction enzyme AluI, and 0.4 kb of AluI-StuI DNA fragment was isolated and purified by agarose gel electrophoresis.

By phosphorylating the 5' terminal of synthetic oligonucleotides [5'd ($_{HO}$CGATAATGGCA$_{OH}$) 3'] and [5'd ($_{HO}$TGCCATTAT$_{OH}$) 3'] by $T_4$ polynucleotide kinase, and it was linked to said 0.4 kb of AluI-StuI DNA fragment by using $T_4$ DNA ligase and then cleaved by restriction enzyme ClaI, and 0.4 kb of ClaI-ClaI DNA fragment was isolated and purified by agarose gel electrophoresis.

On the other hand, vector p TM1 having tryptophan promotor (Imamoto, F., Metabolism, 22, PP 289, 1985) was cleaved by restriction enzyme ClaI, and to this cleavage site, said 0.4 kb of ClaI-ClaI DNA fragment was linked by using $T_4$ DNA ligase, and by using this linkage, the E. coli HB101 strain was transformed, and the E. coli colonies appearing on an LB agar plate containing 50 μg/ml of ampicillin were operated by the boiling method [Maniatis, T. Eritsch E. F. and Sambrook, J, Molecular Cloning, PP 366 (Cold Spring Harbor Laboratory, 1982)] to isolate plasmid, and the size of the cleavage fragment by restriction enzyme was analyzed, and the transformant strain E. coli HB101/p trp IL-2D2-11 containing a desired plasmid p trp IL-2D2-11 was obtained.

This E. coli HB101/p trp IL-2D2-11 was deposited, as Escherichia coli HB101/p trp IL-2D2-11 (FERM BP-1257), in the Fermentation Research Institute Agency of Industrial Science and Technology, No. 1-3, Higashi 1 chome, Yatabe-machi, Tsukuba-gun, Ibaraki-ken 305, Japan, on 23 January, 1987.

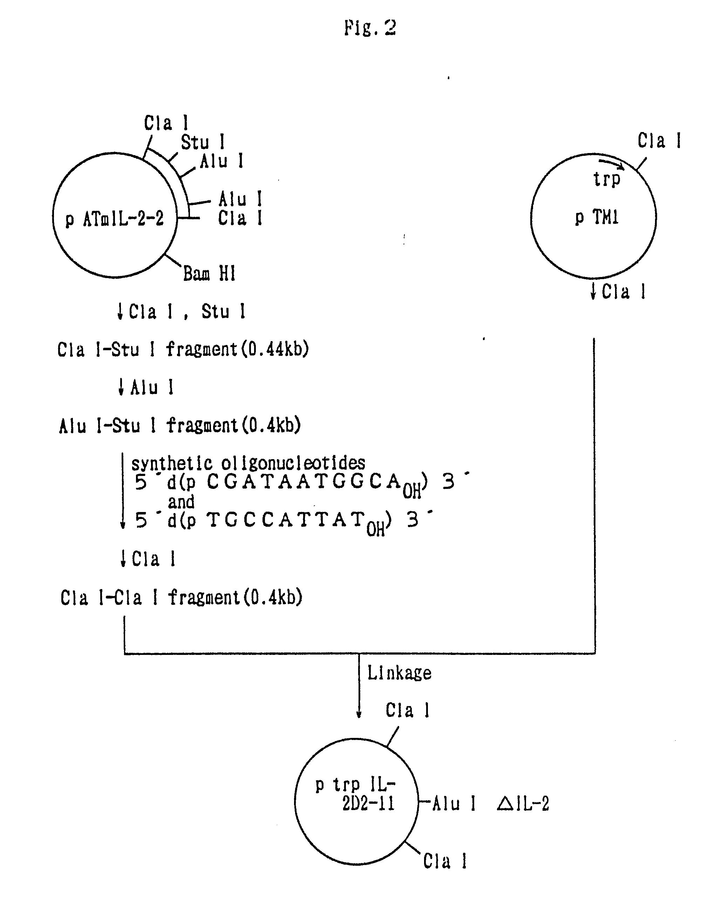

The outline of this process is shown in Fig. 2.

Plasmid p trp IL-2D2-11 is recombinant DNA having the gene to encode the polypeptide lacking the sequence of amino acid numbers 2 to 11, serially numbered from the N terminal of human IL-2 represented by the formula (1) (hereinafter the amino acid numbers refer to the same meaning) coupled downstream of the synthesis initiating codon ATG.

(3)    Cultivation of transformant strain and preparation of desired polypeptide

Escherichia coli HB101/p trp IL-2D2-11 was incubated by shaking overnight at 37°C in 10 ml of LB culture medium containing 50 μm/ml of ampicillin and 20 μg/ml of L-tryptophan, and 1 ml thereof was inoculated in 50 ml of M9 minimum culture medium (0.6% $Na_2HPO_4$, 0.3% $KH_2PO_4$, 0.05% NaCl, 0.1% $NH_4Cl$, 2 mM $MgSO_4$, 0.2% glucose, and 0.1 mM $CaCl_2$) containing 50 μg/ml of ampicillin and 1% casamino acid, and was incubated by shaking at 37°C.  The microorganisms were collected 9 hours later, and were bacteriolyzed in 50 mM TRIS-HCl (pH 8.0), 50 mM EDTA, 15% sucrose, and 1% SDS solution, and a bacterial extractant supernatant was obtained by centrifuging, in which the IL-2 activity was measured.  As a result, the IL-2 activity of $10^4$ units was detected per 1 ml of culture solution of the transformant strain.

(4)    Purification of desired polypeptide

In the above (3), after incubation, the organisms were collected by centrifuging and rinsed once in physiological saline, and were suspended in the same solution, and was frozen and stored at -80°C.  Before use, this frozen product was melted, and an equal volume of 50 mM TRIS- hydrochoride (pH 8.0, containing 50% sucrose) was added, and also 0.1 times by volume of 10 mg/ml lysozyme and 0.1 times by volume of 0.5M EDTA were added, and the solution was let stand for 15 minutes. To this, an equal volume of 50 mM TRIS-hydrochloric acid (pH 8.0, containing 50 mM EDTA and 0.3% Triton X-100) was added, and the mixture was let stand for 15 minutes, and was centrifuged at 3000 rpm for 15 minutes, and the supernatant was removed, and the residual sediment was rinsed three times in the same solution. Furthermore, the sediment was rinsed once in 50 mM TRIS-hydrochloric acid (pH 8.0, containing 150 mM EDTA, 0.3% Triton X-100, and 2M NaCl). Using 50 mM TRIS-hydrochloric acid (pH 8.0, containing 6M urea, 1% Tween 80 and 100 mM $\beta$-mercaptoethanol), this sediment was dissolved, and the eluent fragments were collected by gel filtration column chromatography, using Sephacryl S-300 equilibrated with 50 mM TRIS-hydrochloric acid (pH 8.0, containing 2M urea, 0.2% Tween 80 and 10 mM $\beta$-mercaptoethanol). In succession, after dialyzing with 25 mM sodium acetate (pH 6.0, containing 0.2% Tween 80), fractions were collected by eluting by ion intensity changes of up to 0.2M NaCl by cation exchange column (S Sepharose Fast Flow) chromatography equilibrated with the same solution. These fractions were subjected to Superose 12 gel filtration column chromatography equilibrated with PBS and by collecting the active fraction, polypeptide composed of 123 amino acids lacking the amino acid numbers 2 to 11 of human IL-2 represented by the formula (1) was obtained (polypeptide I).

This polypeptide I was recognized as a single floating band at the position of about 13.5 kilodaltons (kd) as a result of analysis by SDS-PAGE following the method proposed by Laemmli, U. K. et al. (Nature, 277, PP 680, 1970).

Example 2

(1)   Construction of plasmid p trp IL-2Bal

Plasmid p ATm IL-2-1 was cleaved by restriction enzyme ClaI, and about 0.8 kb of DNA fragment containing a region to encode IL-2 protein was isolated and refined by agarose gel electrophoresis.

On the other hand, vector p TM1 having tryptophan promotor was cleaved by restriction enzyme ClaI, and it was linked with said about 0.8 kb of DNA fragment by using T4 DNA ligase, and by this linkage, the E. coli HB101 strain was transformed, and was spread over an LB agar medium containing 50 µg/ml of ampicillin, and the appearing ampicillin resistant strains were operated by boiling method, and plasmid was isolated, and from the restriction maps by various restriction enzymes, E. coli HB101/p trp IL-2D8 possessing a desired plasmid p trp IL-2D8 (about 5.5 kb) was obtained.

After cleaving this plasmid by restriction enzymes BamHI and SalI, about 5.2 kb of BamHI-SalI fragment was isolated and purified by agarose gel electrophoresis.

In addition, by cleaving plasmid p INIII omp Al (The EMBO Journal, 3, PP 2437, 1984) by restriction enzymes BamHI and SalI, about 0.9 kb of BamHI-SalI DNA fragment was isolated and refined by agarose gel elecrophoresis.

These two DNA fragments were linked by T₄ DNA ligase, and the E. coli HB101 strain was transformed, and by the same operation as mentioned above, transformant strain E. coli HB101/p trp IL-2D8T1pp possessing a desired plasmid p trp IL-2D8T1pp (about 6.1 kb) was obtained.

After cleaving this plasmid by restriction enzyme StuI, the double-strand DNA was shaved off by nuclease Bal31, and was cleaved by the restriction enzyme HindIII, and its both ends were treated with DNA polymerase I (Klenow fragment) to form blunt ends , and the ring was closed by T₄ DNA ligase, and the E. coli HB101 strain was transformed, and the transformant strain having a desired plasmid p trp IL-2Bal was obtained in the same manner as mentioned above.

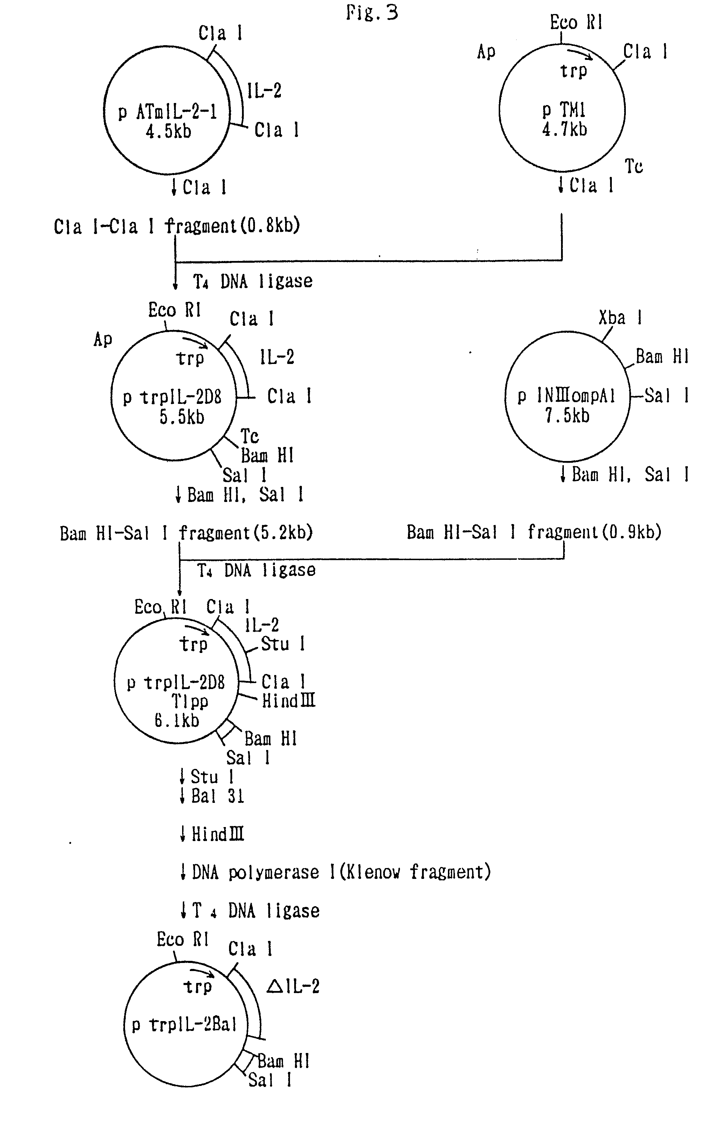

The outline of this operation is shown in Fig. 3.

(2)   Analysis of base sequence of plasmid p trp IL-2Bal

From the transformant strains obtained in (1) (E. coli HB101/p trp IL-2Bal), E. coli HB101 p trp IL-2Bal-7 and E.

coli HB101/p trp IL-2Bal-25 were selected, from which plasmid was isolated and their base sequence were determined. As a result, in both of them, it was found that there was no codon corresponding to the amino acid sequence at the carboxylic terminal side of human IL-2 represented by the formula (1). That is, plasmid p trp IL-2Bal-7 possessed a gene to encode the polypeptide lacking the sequence of amino acid numbers 128 to 133 of human IL-2, while plasmid p trp IL-2Bal-25 had a gene to encode the polypeptide lacking the sequence of 129 to 133, and both of them were supposed to produce a polypeptide having an additional amino acid derived from the base sequence attributable to p BR322, added to said polypeptide.

(3)  Cultivation of transformant strain and preparation of desired polypeptide

The two transformant strains obtained above were respectively cultivated in the same manner as in (3) of Example 1, and bacterial extract supernatants were obtained, in which IL-2 activities were measured. As a result, the IL-2 activity of E. coli HB101/p trp IL-2Bal-7 per 1 ml of culture solution was $3 \times 10^4$ units, and that of E. coli HB101/p trp IL-2Bal-25 was $7.2 \times 10^3$ units.

(4)  Identification of desired polypeptide

As a result of refining the culture supernatant from E. coli HB101/p trp IL-2Bal-7 in the same manner as in (4) of Example 1, the substance showing said activity was identified to be polypeptide having Lys and Leu coupled to the sequence of amino acid numbers 1 to 127 of human IL-2 represented by the formula (1) (that is, Ile of amino acid number 128 of human IL-2 was replaced by Lys, and Ile of amino acid number 129 was replaced by Leu, and the amino acids on and after number 130 were deleted) (polypeptide II).

Similarly, the IL-2 active substance contained in the culture supernatant from E. coli HB101/p trp IL-2Bal-25 was identified to be polypeptide composed of 137 amino acids having the following amino acid sequence coupled to the sequence of amino acid numbers 1 to 128 of human IL-2 represented by the formula (1) (polypeptide III).

| 129 | 130 | 131 | 132 | 133 | 134 | 135 | 136 | 137 |
|-----|-----|-----|-----|-----|-----|-----|-----|-----|
| Ser - | Phe - | Asn - | Ala - | Val - | Val - | Tyr - | His - | Ser |

Claims:

1.  A polypeptide possessing interleukin-2 activities characterized by an amino acid sequence in which at least one amino acid out of the second to eleventh and 128th to 133rd amino acid sequences from the N terminal (Ala) of the amino acid sequence of human interleukin-2 represented by the formula (1)

1

Ala - Pro - Thr - Ser - Ser - Ser - Thr - Lys - Lys -

10

Thr - Gln - Leu - Gln - Leu - Glu - His - Leu - Leu -

20

Leu - Asp - Leu - Gln - Met - Ile - Leu - Asn - Gly -

30

Ile - Asn - Asn - Tyr - Lys - Asn - Pro - Lys - Leu -

40

Thr - Arg - Met - Leu - Thr - Phe - Lys - Phe - Tyr -

50

Met - Pro - Lys - Lys - Ala - Thr - Glu - Leu - Lys -

60

His - Leu - Gln - Cys - Leu - Glu - Glu - Glu - Leu -

70

Lys - Pro - Leu - Glu - Glu - Val - Leu - Asn - Leu -

Ala - Gln - Ser - Lys - Asn - Phe - His - Leu - Arg -

Pro - Arg - Asp - Leu - Ile - Ser - Asn - Ile - Asn -

Val - Ile - Val - Leu - Glu - Leu - Lys - Gly - Ser -

Glu - Thr - Thr - Phe - Met - Cys - Glu - Tyr - Ala -

Asp - Glu - Thr - Ala - Thr - Ile - Val - Glu - Phe -

Leu - Asn - Arg - Trp - Ile - Thr - Phe - Cys - Gln -

Ser - Ile - Ile - Ser - Thr - Leu - Thr      ------ (1)

and the substance having similar effects is deleted or replaced.

2.  A polypeptide possessing interleukin-2 activities according to claim 1, wherein at least one amino acid within the second to the eleventh amino acid sequence from the N terminal (Ala) of amino acid sequence of the formula (1) is deleted or replaced.

3.  A polypeptide possessing interleukin-2 activities according to claim 2, wherein the second to eleventh amino acid sequence from the N terminal (Ala) of amino acid sequence of the formula (1) is deleted.

4. A polypeptide possessing interleukin-2 activities according to claim 2, wherein at least one amino acid within the second to the eleventh amino acid sequence from the N terminal (Ala) of amino acid sequence of the formula (1) is replaced.

5. A polypeptide possessing interleukin-2 activities according to claim 1, wherein at least one amino acid within the 128th to 133rd amino acid sequence from the N terminal (Ala) of amino acid sequence of the formula (1) is deleted or replaced.

6. A polypeptide possessing interleukin-2 activities according to claim 5, wherein at least one amino acid within the 128th to 133rd amino acid sequence from the N terminal (Ala) of amino acid sequence of the formula (1) is deleted.

7. A polypeptide possessing interleukin-2 activities according to claim 5, wherein at least one amino acid within the 128th to 133rd amino acid sequence from the N terminal (Ala) of amino acid sequence of the formula (1) is replaced.

8. A polypeptide possessing interleukin-2 activities according to claim 6, wherein the 128th from the N terminal (Ala) of amino acid sequence of the formula (1) is Lys, the 129th from said N terminal is Leu, and the 130th from said N terminal and subsequent amino acid sequence are deleted.

9.  A polypeptide possessing interleukin-2 activities according to claim 7, wherein the amino acid sequence after the 129th from the N terminal (Ala) of amino acid sequence of the formula (1) is as follows:

| 129 | 130 | 131 | 132 | 133 | 134 | 135 | 136 | 137 |
|-----|-----|-----|-----|-----|-----|-----|-----|-----|
| Ser | Phe | Asn | Ala | Val | Val | Tyr | His | Ser |

10.  A recombinant plasmid incorporating a DNA fragment to encode an amino acid sequence in which at least one amino acid out of the second to eleventh and 128th to 133rd amino acid sequences from the N terminal (Ala) of amino acid sequence of human interleukin-2 represented by the formula (1) and the substance having similar effects is deleted or replaced.

11.  A recombinant plasmid according to claim 10, wherein said DNA fragment is incorporated into downstream of tryptophan promoter.

12.  A recombinant plasmid according to claim 10, wherein the recombinant plasmid is plasmids called p trp IL-2D2-11 and p trp IL-2Bal.

13.  A method of preparation of polypeptide possessing interleukin-2 activities and having an amino acid sequence in which at least one amino acid out of the second to eleventh and 128th to 133rd amino acid sequences from the N terminal (Ala) of the amino acid sequence of human interleukin-2 represented by the formula (1) and the substance having similar effects is deleted or replaced, comprising the steps of cultivating in the culture medium the microorganism transformed by using a recombinant plasmid incorporating a DNA fragment to encode an amino acid sequence in which at least one amino acid out of the second to eleventh and 128th to 133rd amino acid sequences from the N terminal (Ala) of the amino acid sequence of human interleukin-2 represented by the formula (1) and the substance having similar effects is deleted or replaced, forming and accumulating in the culture a polypeptide having the amino acid sequence in which at least one amino acid within the second to eleventh and 128th to 133rd amino acid sequences from the N terminal (Ala) of amino acid sequence of human interleukin-2 represented by the formula (1) and the substance having similar effects is deleted or replaced, and collecting said polypeptide from said culture.

14. A method of preparation of polypeptide possessing interleukin-2 activities and having an amino acid sequence in which at least one amino acid out of the second to eleventh and 128th to 133rd amino acid sequences from the N terminal (Ala) of amino acid sequence of human interleukin-2 represented by the formula (1) and the substance having similar effects is deleted or replaced according to claim 13, wherein said DNA fragment is incorporated downstream of tryptophan promoter.

15. A method of preparation of polypeptide possessing interleukin-2 activities and having an amino acid sequence in which at least one amino acid within the second to eleventh and 128th to 133rd amino acid sequences from the N terminal (Ala) of amino acid sequence of human interleukin-2 represented by the formula (1) and the substance having similar effects is deleted or replaced according to claim 13, wherein the microorganism is one belonging to Escherichia coli.

16. A microorganism containing a recombinant plasmid incorporated a DNA fragment to encode an amino acid sequence in which at least one amino acid out of the second to eleventh and 128th to 133rd amino acid sequences from the N terminal (Ala) of amino acid sequence of human interleukin-2 represented by the formula (1) and the substance having similar effects is deleted or replaced.

17. A microorganism according to claim 16, wherein said DNA fragment is incorporated into downstream of trytophan promotor.

18. A microorganism according to claim 16, wherein the microorganism is one belonging to Eschericha coli.

19. A pharmaceutical preparation comprising a therapeutically effective amount of a polypeptide having an amino acid sequence in which at least one amino acid out of the second to eleventh and 128th to 133rd amino acid sequences from the N terminal (Ala) of human interleukin-2 represented by the formula (1) and the substance having similar effects is deleted or replaced, and a nontoxic carrier.

20. Escherichia coli HB101/p trp IL-2D2-11.

Fig. 1     0233578

Fig. 2

Cla I
Stu I
Alu I

Alu I
Cla I

p ATmIL-2-2

Bam HI

↓Cla I , Stu I

Cla I-Stu I fragment (0.44kb)

↓Alu I

Alu I-Stu I fragment (0.4kb)

synthetic oligonucleotides
5´ d(p C G A T A A T G G C A$_{OH}$) 3´
and
5´ d(p T G C C A T T A T$_{OH}$) 3´

↓Cla I

Cla I-Cla I fragment (0.4kb)

Cla I
trp
p TM1

↓Cla I

Linkage

↓ Cla I

p trp IL-
2D2-11 —Alu I  △IL-2

Cla I

Fig. 3